# EUROPÄISCHE PATENTSCHRIFT

(11) **EP 0 578 145 B1**
(45) Veröffentlichungstag und Bekanntmachung des Hinweises auf die Patenterteilung: **29.10.1997**
(21) Anmeldenummer: 93110573.8
(22) Anmeldetag: 02.07.1993
(51) Int. Cl.: C07C 247/16, C07C 323/25, C07D 213/71, C07D 207/416, C07D 207/404, C07D 207/46, G01N 33/532

(54) **Verbindungen und Verfahren für gezieltes Photoaffinitätslabeling von Biomolekülen**
Compounds and process for photoactivable labelling of biomolecules
Composés et procédé pour le marquage photo-activable de biomolécules

(30) Priorität: 10.07.1992 CH 2179/92
(43) Veröffentlichungstag der Anmeldung: 12.01.1994
(73) Patentinhaber: F. HOFFMANN-LA ROCHE AG, 4002 Basel (CH)
(72) Erfinder: Barner, Richard, CH-4108 Witterswil (CH); Huber, Walter, CH-4303 Kaiseraugst (CH); Hübscher, Josef, CH-4208 Nunningen (CH); Hurst, Jürg, CH-4057 Basel (CH); Schlatter, Daniel, CH-4104 Oberwil (CH)
(74) Vertreter: Buntz, Gerhard

(56) Entgegenhaltungen:
- CHEMICAL ABSTRACTS, vol. 117, 1992, Columbus, Ohio, US; abstract no. 43832z,
- CHEMICAL ABSTRACTS, vol. 109, 1988, Columbus, Ohio, US; abstract no. 3384h,
- CHEMICAL ABSTRACTS, vol. 108, 1988, Columbus, Ohio, US; abstract no. 91133h,

## Beschreibung

Die vorliegende Erfindung betrifft neue, heterotrifunktionelle Verbindungen sowie deren Einsatz in neuen Photoaffinitätslabeling-Verfahren zur gezielten chemischen Funktionalisierung von Biomolekülen, wobei diese neuen Verbindungen auch in herkömmlichen Verfahren des Photoaffinitätslabelings mit Vorteil eingesetzt werden können.

Derartiges Photoaffinitätslabeling wird naturgemäss in wässerigen Medien durchgeführt, in denen die zu markierenden Biomoleküle nicht denaturiert werden. Für die im Photoaffinitätslabeling verwendeten Reagenzien wird deshalb eine genügende Wasserlöslichkeit gefordert. Bei den bisherigen zur Funktionalisierung verwendeten Substanzen handelt es sich vielfach um Verbindungen mit einer nur mässigen Wasserlöslichkeit, sodass sie unter Zuhilfenahme eines organischen Lösungsmittels in die wässerigen Lösungen der Biomoleküle eingetragen werden müssen. Hinzu kommt, dass von diesen Reagenzien im Verlaufe des Labeling-Verfahrens ihre wasserlöslichmachenden Teile abgespalten werden. Durch die Abspaltung der wasserlöslichmachenden Teile besteht die Gefahr, dass die zu markierenden Biomoleküle bei hoher Markierungsrate wasserunlöslich werden. Darüberhinaus werden wasserunlösliche Marker immer die Tendenz zeigen, sich in die hydrophoben Regionen der markierten Moleküle einzulagern und sie werden dadurch für eine nachfolgende Umsetzung schlecht zugänglich werden.

Die in der Literatur beschriebenen Verfahren werden im allgemeinen in homogener Phase durchgeführt [Biochemistry, 1980, 19, 2196-2203; Biochimica et Biophysica Acta, 1983, 761, 152-162]. Dabei wird zuerst einer der Partner eines Affinitätskomplexes (z.B. Antigen beim Labeling von Antikörpern, oder Ligand beim Labeling von Rezeptoren) mit der chemisch reaktiven Gruppe des heterotrifunktionellen Reagenzes zur Reaktion gebracht. Bei einem grossen Bindungspartner (z.B. Protein-Protein-Affinitätskomplex) muss, um ein effizientes Affinitätslabeling zu erreichen, dieser Partner des Affinitätskomplexes mit mehreren heterotrifunktionellen Molekülen reagiert haben. Nach dieser chemischen Modifikation des einen Bindungspartners wird der Affinitätskomplex durch Zugabe des zweiten Partners gebildet. Durch photolytische Aktivierung der photoaktivierbaren Gruppen kann dieser Komplex durch kovalente Bindungen stabilisiert werden. Da es sich bei diesen Gruppen in der aktivierten Form um hochreaktive Gruppen handelt, welche augenblicklich mit ihrer nächsten Umgebung abreagieren, erfolgt diese Bindungsbildung ausschliesslich an den Kontaktstellen (oder in der Nähe dieser Kontaktstellen) der beiden Partner des Affinitätskomplexes. Dieser Affinitätskomplex kann anschliessend wieder gelöst werden, indem die dritte funktionelle Gruppe -die Disulfidgruppe- durch Reduktion gespalten wird. Durch geeignete Veränderung des pH-Wertes oder der Ionenstärke können nach dieser Reduktion die beiden Partner freigesetzt werden, wobei beide über freie Thiolgruppen verfügen; bei dem einen sind diese willkürlich über die Oberfläche verteilt, während sie beim andern Partner auf die Regionen in oder in der Nähe der Bindungsstelle des Affinitätskomplexes lokalisiert sind. In einem nachfolgenden Trennschritt müssen die beiden Partner unter Zuhilfenahme bekannter chromatographischer Techniken getrennt werden.

B.J.M. Thevenin, 1992, Eur. J. Biochem. 206, 471-477, K. Muramoto, 1988, Agric. Biol. Chem. 52, 547-554 and E.G. Shephard, 1988, Anal. Biochem. 168, 306-313 beschreiben solche heterotrifunktionelle Photoaffinitätslabelingreagenzen.

Die Alkylthiolgruppen ermöglichen einerseits ein kovalentes Verankern der in dieser Weise modifizierten Biomoleküle auf entsprechend funktionalisierten Oberflächen, andererseits das Anfügen zusätzlicher Gruppen z.B. fluorophorer Gruppen, wobei sowohl der elektrophile Charakter der Thiolgruppe wie auch ein Thiolaustausch für solche Umsetzungen herangezogen werden können. Der Alkylteil des Thiols hat die Funktion eines Spacers.

Der hier vorliegenden Erfindung liegt die Aufgabe zugrunde, Verbindungen für das Affinitätslabeling von Biomolekülen bereitzustellen, welche vor und nach der Markierung der Biomoleküle wasserlöslichmachende Gruppen besitzen, sodass die markierten Biomoleküle ihre Wasserlöslichkeit bewahren und die Markierungsmoleküle nach der Verankerung auf der Oberfläche der Biomoleküle bevorzugt frei in die Lösung ragen.

Ferner liegt der Erfindung die Aufgabe zugrunde die in bekannten Verfahren erforderliche chromatographische Trennung zu vermeiden.

Die Aufgabe wird gelöst durch Verbindungen der allgemeinen Formel I, sowie durch ein neues Verfahren, welches sich dadurch auszeichnet, dass der für die gezielte Markierung eines Biomoleküls beigezogene Partner des Affinitätskomplexes auf einer Festphase kovalent immobilisiert ist und dadurch aufwendige chromatographische Trennverfahren wegfallen.

Die neuen heterotrifunktionellen Verbindungen gemäss vorliegender Erfindung sind Verbindungen der allgemeinen Formel: 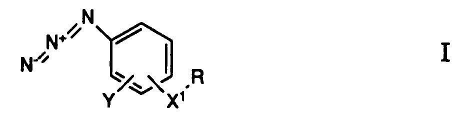 worin X¹ eine Carbonyl- ( >C=O) oder Sulfonylgruppe ( >SO₂) und Y=H,Y' oder X¹-Y' bedeutet. Y' ist eine Hydroxy- bzw. Alkoxygruppe ( -O-Y'') oder eine Aminogruppe ( - NH-Y''), dabei bedeutet Y''=H oder eine wasserlöslichmachende Gruppe vom Typ (CH₂)ₙA. n=1-6

Dabei ist A ein Glykol- bzw. Oligoethylenglykol-Substituent, eine tert. bzw. quaternäre Aminogruppe wie Pyridyl, Dialkylamino bzw. N-Alkylpyridinium, Trialkylammonium. Alkyl bedeutet einen niederen Alkylrest ca. C₁ - C₄.

Die Gruppe R ist eine funktionelle Gruppe der allgemeinen Formel: 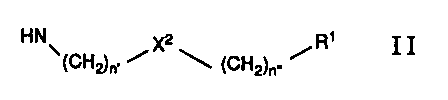 worin X² eine Disulfid- (-S-S-) oder Methylengruppe (-CH₂-) bedeuten.

R¹ bedeutet ein Amino- (-NH-R²) oder Carboxylderivat (-CO-R³). Dabei bedeutet R²=H oder eine derivatisierte Carboxyalkanoylgruppe (-CO-(CH₂)ₙCO-R³).

CO-R³ ist eine aktivierte Carboxylgruppe wie z.B. ein Säurehalogenid, Imidazolid, Hydrazid, Anhydrid, eine mit einer Dithiopyridylgruppe (-NH-(CH₂)ₙ'''-S-S-Pyridyl) derivatisierte Carboxylgruppe oder ein reaktiver Ester mit z.B. Hydroxysuccinimid, Isoharnstoff, Hydroxysuccinimidsulfonsäure.
n',n'',n'''=1-6.

Wenn X² eine Methylengruppe bedeutet, muss R³ eine Disulfidgruppe enthalten, wobei R³ z.B. ein Cystaminderivat -NH-(CH₂)₂-S-S-(CH₂)₂-NH-R² bedeutet, worin R² die obenstehende Bedeutung hat und das in R² enthaltene R³ keine Disulfidgruppe enthält.

Für Y=H ist X¹-R ( ohne R³ ) hydrophil ( z.B. X¹=-SO₂- oder R¹=tert. Amin oder quartäres Ammonium).

Für hydrophiles X¹-R ( ohne R³ ) kann Y gewünschtenfalls auch X¹-R sein (doppelte Verankerung).

Die Aminogruppe R¹ kann auch in andere reaktive Gruppen umgewandelt werden wie in ein Isocyanat, Isothiocyanat, Vinylsulfonsäureamid (-NH-SO₂-CH=CH₂), Maleinimid, halogensubstituierte Triazinamino- , Pyrimidinamino- oder Pyridinaminoverbindungen (z.B. Dichlortriazin), 2-Halogencarbonsäurederivate (z.B. mit 2-Halogenessigsäurehalogenid, 2-Halogenpropionsäurehalogenid und dergleichen), Monoamide aus Dicarbonsäuredihalogeniden, Epoxide z.B. mit Epichlorhydrin oder ein Cyclohexendionderivat via Michaeladdition an ein Chinon.

| | | |
|---|---|---|
| X¹ = -CO- | | |
| X¹ = -SO₂- | | |
| X² = -S-S- | | |
| X² = -(CH₂)- | | |
| Y = -H | | A = -O-(CH₂)₂-O-H |
| Y = -Y' | | A = -O-[(CH₂)₂-O]ₙ-H |
| Y= -CO-Y' = -X¹-Y' | Y' = -O-Y'' Y'' = -H | A = -N(Alkyl)₂ |
| Y = -SO₂-Y' = -X¹-Y' | Y' = -NH-Y''X'' = -(CH₂)ₙ-A | A = -N⁺(Alkyl)₃ |
| | | A = -Pyridin |
| | | A = -Pyridinium(N-Alkyl) |
| R¹ = -CO-R³ | R² = H | COR³ = CO-Cl |
| R¹ = -NH-R² | R² = -CO-(CH₂)ₙ-CO-R³ | COR³ = CO-O-Acyl |
| | | COR³ = CO-Isoharnstoff |
| | | COR³ = CO -OSu |
| | | COR³ = CO -OSu(SO₃H) |
| | | COR³ = CO -NH-NH₂ |
| | | COR³ = CO -NH-(CH₂)ₙ'''-S-S-Pyridyl |
| | | COR³ = CO-NHCONH₂ |
| | | COR³ = CO-Imidazolyl |

X² = -CH₂-,
R¹ = COR³ = CO-NH-(CH₂)₂-S-S-(CH₂)₂-NH-R² (R² = H, CO-(CH₂)ₙ-CO-R³) wobei R³ wie in Spalte 3 definiert ist mit Ausnahme der Disulfidverb.)
R¹ = Pyridinium(N-CH₂-CO-R³)
   - R¹ = NH₂ ⇒: -N=C=O
   -N=C=S
   CH₂=CH-SO₂-NH-
   -Maleinimidyl
   -NH-CO-CH(Cl)-Alkyl
   -NH-CO-Alkylen-CO-Cl
   -NH-CH₂-Oxiran
   -NH-Cyclohexendion
   -NH-Dichlortriacinyl

Diese neuen Verbindungen sind heterotrifunktionell, weil sie über eine chemisch reaktive Gruppe R, eine photochemisch reaktive Gruppe Ar-N₃ und eine reduktiv spaltbare -SS-(X₂) Gruppe verfügen. Die chemisch reaktive Gruppe und die photochemisch reaktive Gruppe sind durch eine Spacergruppe voneinander getrennt, wobei diese Spacergruppe die spaltbare -SS-Gruppe beinhaltet. Wasserlöslichmachende Gruppen sind z.B. Carbon- oder Sulfonsäuren oder Derivate davon, welche entweder am aromatischen Ring oder an der Spacergruppe zwischen aromatischem Ring und der spaltbaren - -SS-Gruppe angebracht sind. Durch diese Platzierung wird sichergestellt, dass diese wasserlöslichmachenden Gruppen mit dem Marker in die zu markierenden Biomoleküle eingebracht werden.

Das neue, an einer Festphase erfolgende Verfahren zur gezielten Einführung von Alkylthiolgruppen in Biomoleküle, benutzt zur Zielerreichung eine intermediäre, ausgerichtete Fixierung der zu markierenden Biomoleküle an der Oberfläche der Festphase. Die Oberfläche ist so modifiziert, dass dieses intermediäre Fixieren über die zu markierenden Molekülteile erfolgt. Dies kann z.B. dadurch erreicht werden, dass auf dieser Oberfläche der Partner eines Affinitätskomplexes verankert ist, welcher die zu markierenden Molekülbereiche erkennt (z.B. Protein A für die Markierung von Fc-Teilen von Antikörpern, Antigene oder Liganden für die Markierung von Antigenbindenden oder Ligand-bindenen Bereichen von Antikörpern oder von Rezeptoren und umgekehrt oder sDNA und DNA für die Markierung von DNA-bindenden Bereichen von DNA-bindenden Proteinen, etc.), wobei an diese immobilisierten Partner des Affinitätskomplexes das heterotrifunktionelle Reagenz unter Zuhilfenahme der chemisch reaktiven Gruppe gebunden ist. Nach der ausgerichteten Fixierung der zu markierenden Moleküle an dieser Oberfläche werden diese Moleküle durch eine lichtinduzierte Reaktion (Aktivierung der photoreaktiven Gruppe) an der Oberfläche gebunden. Es zeigt sich nun, dass die oben vorgestellten wasserlöslichen, heterotrifunktionellen Reagenzien für das Fixieren an der Oberfläche besonders geeignet sind. Durch die spezielle Platzierung der wasserlöslichmachenden Gruppen (am Phenylring oder in der Spacergruppe zwischen Phenylgruppe und -SS-Funktion) ragt die photochemisch aktive Region des Moleküls nach der Fixierung auf der Oberfläche in die wässerige Phase; d.h. die photoreaktive Gruppe befindet sich in der Nachbarschaft des zu markierenden Moleküls und es wird dadurch die Wahrscheinlichkeit einer Kopplung sehr stark erhöht. Dieses Binden erfolgt hier bevorzugt über die oberflächennahen Strukturteile der zu markierenden Verbindung, bzw. über diejenigen Regionen, welche an der Ausbildung des Affinitätskomplexes beteiligt sind. Nach Reduktion der Disulfidbindung mit z.B. Dithioeritrol können die markierten Moleküle bei geeignetem pH-Wert oder Ionenstärke oder unter Zugabe von Detergenzien von der Oberfläche der Festphase abgewaschen werden.

Erfindungsgemäss ist es nicht zwingend nötig, dass der auf der Oberfläche der Festphase immobilisierte Partner des Affinitätskomplexes mit dem photoaktivierbaren Reagenz umgesetzt wird. Diese reaktive Gruppe kann auch über ein drittes Molekül auf die Oberfläche gebracht werden, welches an diese Oberfläche vor oder gleichzeitig mit dem zu immobilisierenden Partner des Affinitätskomplexes immobilisiert werden kann. Der Vorteil des Einbezugs eines solchen markierten dritten Moleküls liegt darin, dass dadurch der kovalent immobilisierte Partner des Affinitätskomplexes nicht mehr markiert werden muss und dadurch auch kein Aktivitätsverlust dieses Partners eintritt. Bei diesem Labeling-Verfahren werden nicht nur die an der Bildung des Affinitätskomplexes beteiligten Bereiche der zu markierenden Moleküle markiert, sondern alle Bereiche der Moleküle, welche durch die Ausrichtung in die Nähe der Oberfläche zu liegen kommen.

Erfindungsgemäss zeigt dieses Verfahren darüberhinaus auch den Vorteil, dass beim Arbeiten an einer Oberfläche für das Ausrichten der zu markierenden Biomoleküle nicht zwingend der Partner eines Affinitätskomplexes herangezogen werden muss. Für das Ausrichten bestimmter Typen von Biomolekülen kann nämlich die Oberfläche einer Festphase chemisch so präpariert werden, dass ein gegebenes, zu markierendes Molekül nur über ganz bestimmte Bereiche an diese Oberfläche adsorbiert und demzufolge diese Bereiche markiert werden, ohne dass dabei ein Partner eines Affinitätskomplexes herangezogen wird (z.B. Membran-analoge Oberfläche für die Markierung eines Transmembranteils oder Bereiche in der Nähe dieses Transmembranteils eines Membranproteins oder Metallkomplexe an der Oberfläche mit ungesättigter Koordinationssphäre, welche durch bestimmte Aminosäuresequenzen eines Proteins abgesättigt werden für die Markierung des Proteins in der Nähe dieser Sequenz etc.). Zusätzlich zu dieser ausrichtenden Funktion ist die Oberfläche mit den heterotrifunktionellen Verbindungen umgesetzt. Die photoreaktiven Bereiche dieser Verbindungen ragen auch hier aufgrund ihrer Wasserlöslichkeit in die wässerige Phase und reagieren nach der Aktivierung bevorzugt mit den oberflächennahen Bereichen der zu markierenden Moleküle.

Das folgende Beispiel erläutert die Herstellung der heterotrifunktionellen Verbindungen:

41mg N-(p-Azidoberzolsulfonyl) N'-(3-carboxypropionyl)cystamin wurden 5 Stunden gerührt mit 1ml Thionylchlorid und anschliessend im WSV eingeengt. Das rohe Säurechlorid wurde in 5ml THF gelöst und mit 14mg N-Hydroxysuccinimid als Lösung in 1ml Pyridin versetzt. Es wurde 2 Stunden gerührt und anschliessend im HV eingeengt. Man erhielt 68mg N-(p-Azidobenzolsulfonyl) N'-(3-succinimidyloxycarbonylpropionyl) cystamin als Pyridiniumsalz.

Das verwendete Ausgangsmaterial wurde wie folgt hergestellt:

2,2g Cystamindihydrochlorid wurden in 20ml Wasser gelöst und mit NaOH auf pH10 eingestellt. In dieser Lösung wurden 2,1g p-Azidobenzolsulfochlorid suspendiert und 5 Stunden bei Raumtemperatur gerührt. Das ausgefallene N-(p-Azidobenzolsulfonyl)cystamin wurde mit 2g Bernsteinsäureanhydrid umgesetzt und über Nacht gerührt. Die dabei entstandene Lösung wurde mit HCl angesäuert, anschliessend filtriert und mit Wasser gewaschen. Der Rückstand wurde bei RT im HV getrocknet und ergab 1,53g N-(p-Azidobenzolsulfonyl)-N'-(3-carboxypropionyl)cystamin. Das IR zeigte Banden bei 3283(Amid-NH) 2134(Azid), 1714(Säurecarbonyl), 1650(Amid), 1589+1547 (Aromat), 1284(COOH), 1328+1180(Arylsulfonyl), 839(p-disubst.Benzol).
DC(Kieselgel-NH₃ conc./EtOH = 1%)
Rf = 0,7. Smp:Zers. bei 163°.

## Patentansprüche

1. Heterotrifunktionelle Verbindungen der allgemeinen Formel 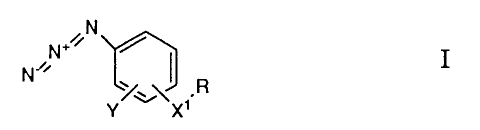 worin X¹ eine Carbonyl- oder Sulfonylgruppe bedeutet; Y bedeutet Wasserstoff, Y' oder X¹Y', wobei X¹ die oben erwähnte Bedeutung hat und Y' eine Hydroxygruppe, eine Aminogruppe oder eine Alkoxygruppe bedeutet; R bedeutet eine funktionelle Gruppe der allgemeinen Formel: 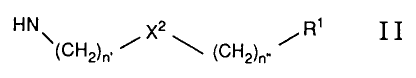 worin X² eine Disulfid- oder Methylengruppe bedeuten;
R¹ bedeutet eine Aminogruppe (-NH-R²), wobei R² Wasserstoff oder eine derivatisierte Carboxylalkanoylgruppe bedeutet; R¹ bedeutet auch eine durch Umsetzung der Aminogruppe entstandene reaktive Gruppe die aus einem Isocyanat, Isothiocyanat, Vinylsulfonsäureamid (-NH-SO₂-CH = CH₂), Maleinimid, halogensubstituierte Triazinamino-, Pyrimidinamino- oder Pyridinaminoverbindungen, 2-Halogencarbonsäureamiden, Monoamiden aus Dicarbonsäuredihalogeniden, Epoxiden durch Umsetzung mit Epichlorhydrin (-NH-CH₂-Oxiran), Cyclohexendionderivaten (-NH-Cyclohexendion) durch Michaeladdition an ein Chinon ausgewählt ist; R¹ bedeutet ferner ein Carboxylderivat (CO-R³), wobei CO-R³ ein Säurehalogenid, Imidazolid, Hydrazid, Anhydrid, eine mit einer Dithiopyridylgruppe (-NH-(-CH₂)ₙ'''-S-S-Pyridyl) derivatisierte Carboxylgruppe oder ein reaktiver Ester mit Hydroxysuccinimid, Isoharnstoff, Hydroxysuccinimid-sulfonsäure beteutet;
mit der Massgabe wenn X² eine Methylengruppe bedeutet muss R¹ eine Disulfidgruppe enthalten; n',n'',n''' stehen für ganze Zahlen von 1-6.

2. Verbindungen nach Anspruch 1, worin die Amino- oder Alkoxygruppe der oben erwähnten Gruppe Y' eine Gruppe -NH-Y'' oder -OY'' ist, wobei Y'' Wasserstoff oder eine Gruppe vom Typ-(CH₂)ₙA bedeutet, wobei A ein Glykol- bzw. Oligoethylenglykol-Substituent, eine tert. bzw. quaternäre Aminogruppe wie Pyridyl, Dialkylamino bzw. N-Alkylpyridinium, Trialkylammonium bedeutet; n steht für eine ganze Zahl von 1-6.

3. Photoaffinitätslabeling-Verfahren zur gezielten Einführung von Alkylthiolgruppen, dadurch gekennzeichnet, dass die zu markierenden Biomoleküle an eine ausrichtende Oberfläche physisorbiert werden, und diese Oberfläche gleichzeitig photoaktivierbare, kovalent gebundene Moleküle der Formel I trägt.

4. Photoaffinitätslabeling-Verfahren nach Anspruch 3, dadurch gekennzeichnet, dass die ausgerichteten, physisorbierten Moleküle in einer lichtinduzierten Reaktion an den photoaktivierbaren Rest der auf der Oberfläche gebundenen Moleküle der Formel I kovalent gebunden werden.

5. Verfahren nach einem der Ansprüche 3 bis 4, dadurch gekennzeichnet, dass nach der photochemischen Verknüpfung die Disulfidbrücke in den Verbindungen der allgemeinen Formel I reduktiv gespalten wird.

6. Verfahren nach einem der Ansprüche 3 bis 5, dadurch gekennzeichnet, dass nach der Spaltung der Disulfidbrücke die Bindung der Biomoleküle an die Oberfläche gelöst wird.

7. Verwendung von Verbindungen der allgemeinen Formel I in Photoaffinitätslabeling-Verfahren.

## Claims

1. Heterotrifunctional compounds of the general formula 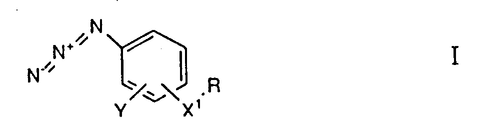 wherein X¹ signifies a carbonyl or sulphonyl group; Y signifies hydrogen, Y' or X¹Y' in which X¹ has the aforementioned significance and Y' signifies a hydroxy group, an amino group or an alkoxy group; R signifies a functional group of the general formula: 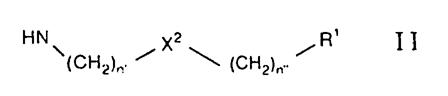 wherein X² signifies a disulphide or methylene group;
R¹ signifies an amino group (-NH-R²) in which R² signifies hydrogen or a derivatized carboxylalkanoyl group; R¹ also signifies a reactive group resulting by reaction of the amino group, selected from an isocyanate, isothiocyanate, vinylsulphonamide (-NH-SO₂-CH = CH₂), maleimide, halo-substituted triazinoamino, pyrimidinamino or pyridinamino compounds, 2-halocarboxamides, monoamides from dicarboxylic acid dihalides, epoxides by reaction with epichlorohydrin (-NH-CH₂-oxirane), cyclohexenedione derivatives (-NH-cyclohexenedione) by Michael addition to a quinone; R¹ further signifies a carboxyl derivative (CO-R³) in which CO-R³ signifies an acid halide, imidazolide, hydrazide, anhydride, a carboxyl group which is derivatized with a dithiopyridyl group (-NH-(-CH₂)ₙ'''-S-S-pyridyl) or a reactive ester with hydroxysuccinamide, isourea, hydroxysuccinimidesulphonic acid;
with the proviso that, when X² signifies a methylene group, R¹ must contain a disulphide group; n', n'', n''' stand for integers of 1-6.

2. Compounds according to claim 1, wherein the amino or alkoxy group of the aforementioned group Y' is a group -NH-Y'' or -OY'' in which Y'' signifies hydrogen or a group of the type -(CH₂)ₙA where A signifies a glycol or oligoethylene glycol substituent, a tert. or quaternary amino group such as pyridyl, dialkylamino or N-alkylpyridinium, trialkylammonium; and n stands for an integer of 1-6.

3. A photoaffinity labelling method for the planned introduction of alkylthio groups, characterized by physically adsorbing the biomolecule to be labeled on an oriented surface which simultaneously carries photoactivatable, covalently bonded molecules of formula I.

4. A photoaffinity labeling method according to claim 3, characterized in that the oriented, physically absorbed molecules are covalently bonded in a light-induced reaction on the photo-activatable residue of the molecules of formula I bonded to the surface.

5. A method according to claim 3 or 4, characterized in that the disulphide bridge in the compounds of general formula I is reductively cleaved after the photochemical linkage.

6. A method according to any one of claims 3 to 5, characterized in that the bonding of the biomolecule to the surface is released after the cleavage of the disulphide bridge.

7. The use of compounds of general formula I in a photoaffinity labeling method.

## Revendications

1. Composés hétérotrifonctionnels de formule générale: 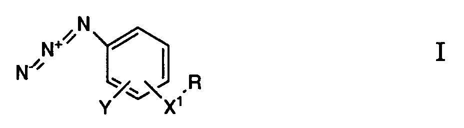 dan laquelle X¹ signifie un groupe carbonyle ou sulfonyle; Y signifie l'hydrogène, Y' ou X¹Y', X¹ ayant la signification ci-dessus et Y' signifiant un groupe hydroxy, un groupe amino ou un groupe alkoxy; R signifie un groupe fonctionnel de formule générale: 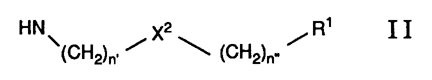 dans laquelle X² signifie un groupe disulfure ou méthylène;
R¹ signifie un groupe amino (-NH-R²) dans lequel R² signifie l'hydrogène ou un groupe carboxylalkanoyle dérivé ; R¹ signifie aussi un groupe réactif formé par réaction du groupe amino, lequel est choisi parmi l'isocyanate, l'isothiocyanate, le vinylsulfonamide (-NH-SO₂-CH = CH₂), le maléimide, des composés halo-substitués triazinoamino, pyrimidinamino ou pyridinamino, des 2-haloamides, des monoamides de dihalogénures d'acides dicarboxyliques, des époxides par réaction avec l'épichlorhydrine (-NH-CH₂-oxiranne), des dérivés de cyclohexènedione (-NH-cyclohexènedione) par addition Michael sur une quinone; R¹ signifie de plus un dérivé de carboxyle (CO-R³), CO-R³ signifiant un halogénure d'acide, un imidazolide, un hydrazide, un anhydride, un dérivé de carboxyle dérivé avec un groupe dithiopyridyle (-NH-(-CH₂)ₙ'''-S-S-pyridyl) ou un ester réactif avec l'hydroxysuccinimide, l'isourée, l'acide hydroxysuccinimidesulfonique;
avec la condition que lorsque X² signifie un groupe méthylène, R¹ doit contenir un groupe disulfure; n', n'', n''' sont des nombres entiers de 1 à 6.

2. Composés selon la revendication 1, dans lesquels le groupe amino ou alkoxy du groupe Y' mentionné ci-dessus est un groupe -NH-Y'' ou -OY'', Y'' signifiant l'hydrogène ou un groupe du type (CH₂)ₙA, dans lequel A est un substituant glycol ou oligoéthylèneglycol, un groupe amino tertiaire ou quaternaire tel que le pyridyle, un dialkylamino ou un N-alkylpyridinium, un trialkylammonium; n est un nombre entier de 1 à 6.

3. Procédé de marquage par photoaffinité pour l'introduction contrôlée de groupes alkylthiol, caractérisé en ce que les biomolécules à marquer sont adsorbées physiquement sur une surface orientée, qui porte simultanément des molécules liées de façon covalente de formule I.

4. Procédé de marquage par photoaffinité selon la revendication 3, caractérisé en ce que les biomolécules orientées, adsorbées physiquement sont liées de façon covalente dans une réaction photoinduite au reste photoactivable des molécules de formule I liées à la surface.

5. Procédé selon la revendication 3 ou 4, caractérisé en ce qu'après le couplage photochimique le pontage disulfure dans les composés de formule générale I est séparé par réduction.

6. Procédé selon l'une des revendications 3 à 5, caractérisé en ce qu'après la séparation du pontage disulfure la liaison des biomolécules à la surface est supprimée.

7. Utilisation des composés de formule générale I dans un procédé de marquage par photoaffinité.
